# EUROPEAN PATENT SPECIFICATION

(11) **EP 3 523 307 B1**
(45) Date of publication and mention of the grant of the patent: **17.11.2021**
(21) Application number: 17857985.0
(22) Date of filing: 06.10.2017
(51) Int. Cl.: C07D 495/00

(54) **PROCESS FOR THE PREPARATION OF DORAOLZMIDE HYDROCHLORIDE**
VERFAHREN ZUR HERSTELLUNG VON DORAOLZMID-HYDROCHLORID
PROCÉDÉ DE PRÉPARATION DU CHLORHYDRATE DE DORZOLAMIDE

(30) Priority: 07.10.2016 IN 201621034446
(43) Date of publication of application: 14.08.2019
(73) Proprietor: Indoco Remedies Limited, Mumbai 400 098 MAH (IN)
(72) Inventor: SHETH, Nilima, Navi Mumbai Maharashtra 400701 (IN); KUDUVA, Srinivasan Subramanian, Navi Mumbai Maharashtra 400701 (IN); RAMESAN, Palangat Vayalileveetil, Navi Mumbai Maharashtra 400701 (IN); PANANDIKAR, Aditi Milind, Mumbai Maharashtra 400098 (IN)
(74) Representative: Forrest, Stuart
(86) International application number: PCT/IN2017/050453
(87) International publication number: WO 2018/066004

(56) References cited:
- WO-A1-2010/061398
- WO-A1-2011/101704
- US-A1- 2006 142 595
- S.M. THOMAS ET. AL.: "In Process Tests for the Synthesis of Dorzolamide", ANALYTICAL CHEMISTRY, vol. 2, 1 January 1997 (1997-01-01), pages 432-439, XP002903039,

## Description

### FIELD OF INVENTION:

The present invention relates to an improved process for the preparation of dorzolamide hydrochloride.

### BACKGROUND OF THE INVENTION:

Dorzolamide hydrochloride is a carbonic anhydrase (CA) inhibitor. It is chemically represented by (4S,6S)-4-(ethylamino)-6-methyl-5,6-dihydro-4H-thieno[2,3-b]thiopyran-2-sulfonamide 7,7-dioxide hydrochloride; and is structurally represented by Formula I.

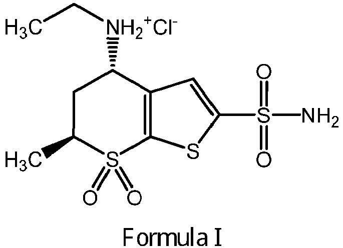

It acts as an anti-glaucoma agent, in open-angle glaucoma and ocular hypertension. It is used in ophthalmic solutions to lower intraocular pressure (IOP).

The compound dorzolamide hydrochloride has been in the market for very long time. It is administered as a topical ophthalmic in the form of a solution and marketed under the brand name T rusopt.

Dorzolamide hydrochloride and process for its preparation are first described in the patent, US 4,797,413 (US 413 Patent) and its corresponding European patent, EP 0296879. The process described in US 413 patent involves reacting thiophene-2-thiol with but-2-enoic acid and further proceeds with formation of racemic 4-(ethylamino)-6-methyl-5,6-dihydro-4H-thieno[2,3-b]thiopyran-2-sulfonamide 7,7-dioxide (dorzolamide base).

A number of further processes for the preparation of dorzolamide hydrochloride have been devised and in many of these, as well as in the above US413 Patent, the last step of the process involves the removal of diastereomeric impurity from the racemic mixture of dorzolamide base. To obtain pure dorzolamide hydrochloride devoid of the diastereomeric impurity of cis-isomer from the racemic compound, as per the patent US 413, racemic mixture of dorzolamide base is subjected to column chromatography and then resolution is carried out using resolving agent di-p-toluoyl-L-tartaric acid monohydrate in n-propanol. The salt formed is treated with base to get dorzolamide free base, which is reacted with ethanolic hydrochloric acid to get dorzolamide hydrochloride. The compound is further recrystallised from mixture of solvents viz., methanol and isopropanol to get pure dorzolamide hydrochloride.

US 5,688,968 describes a process for preparation of dorzolamide hydrochloride, wherein chiral hydroxyl sulfone compound having fixed chirality, proceeds via Ritter reaction to obtain dorzolamide base having mixture of cis- and trans-isomer. The compound dorzolamide base is reacted with maleic acid to isolate maleate salt of dorzolamide. The salt is again converted to free base and then reacted with hydrochloric acid in ethyl acetate to get required pure trans-isomer of dorzolamide hydrochloride.

The PCT patent publication WO2006038222 discloses the preparation of dorzolamide hydrochloride, wherein the cis- and trans-isomer of racemic dorzolamide base is separated using resolution via chiral salt formation with dibenzoyl-L-tartaric acid monohydrate or di-p-toluoyl-L-tartaric acid monohydrate in methanol which on neutralization results in dorzolamide base. The base is then reacted with hydrochloric acid in isopropanol to give dorzolamide hydrochloride which is recrystalised in isopropanol to obtain pure dorzolamide hydrochloride.

Another US patent US 7,109,353 discloses the process for preparation of dorzolamide hydrochloride, wherein racemic 4-(ethylamino)-6-methyl-5,6-dihydro-4H-thieno[2,3-b]thiopyran-2-sulfonamide 7,7-dioxide is treated with mineral acid to form the corresponding salt, which is then converted to racemic trans-4-(ethylamino)-6-methyl-5,6-dihydro-4H-thieno[2,3-b]thiopyran-2-sulfonamide 7,7-dioxide and resolved with di-p-toluoyl-D-tartaric acid followed by neutralization of the chiral salt to isolate trans-(4S,6S)-4-(ethylamino)-6-methyl-5,6-dihydro-4H-thieno[2,3-b]thiopyran-2-sulfonamide 7,7-dioxide. The compound trans-(4S,6S)-4-(ethylamino)-6-methyl-5,6-dihydro-4H-thieno[2,3-b]thiopyran-2-sulfonamide 7,7-dioxide on reaction with hydrochloric acid in ethanol results in required trans-dorzolamide hydrochloride.

PCT patent publication WO2007122130 discloses the process for preparation of (4S,6S)-4-(ethylamino)-6-methyl-5,6-dihydro-4H-thieno[2,3-b]thiopyran-2-sulfonamide 7,7-dioxide, wherein racemic 4-(ethylamino)-6-methyl-5,6-dihydro-4H-thieno[2,3-b]thiopyran-2-sulfonamide 7,7-dioxide having trans:cis diastereomeric mixture of 80:20 is treated with maleic acid in acetone to isolate racemic trans-4-(ethylamino)-6-methyl-5,6-dihydro-4H-thieno[2,3-b]thiopyran-2-sulfonamide 7,7-dioxide maleate salt having trans:cis diastereomeric mixture of 95:5. The isolated maleate salt is then treated with base and reacted with (1S)-(+)-10-camphorsulfonic acid to get corresponding (4S,6S)-4-(ethylamino)-6-methyl-5,6-dihydro-4H-thieno[2,3-b]thiopyran-2-sulfonamide 7,7-dioxide (1S)-(+)-10-camphorsulfonate salt, which is neutralized to give pure (4S,6S)-4-(ethylamino)-6-methyl-5,6-dihydro-4H-thieno[2,3-b]thiopyran-2-sulfonamide7,7-dioxide.

PCT patent publication WO2008135770 discloses the process for the preparation of dorzolamide hydrochloride, wherein the racemic 4-(ethylamino)-6-methyl-5,6-dihydro-4H-thieno[2,3-b]thiopyran-2-sulfonamide 7,7-dioxide is treated with carboxylic acid selected from the group consisting of fumaric acid, benzoic acid, acetic acid, salicylic acid and p-hydroxybenzoic acid, which selectively forms an acid addition salt with the trans-isomer and removes the undesirable cis-isomer from the mixture of cis and trans-isomers. The trans-4-(ethylamino)-6-methyl-5,6-dihydro-4H-thieno[2,3-b]thiopyran-2-sulfonamide 7,7-dioxide acid addition salt is converted to trans-(ě)-4-(ethylamino)-6-methyl-5,6-dihydro-4H-thieno[2,3-b]thiopyran-2-sulfonamide 7,7-dioxide by conventional methods. The compound trans-(ě)-4-(ethylamino)-6-methyl-5,6-dihydro-4H-thieno[2,3-b]thiopyran-2-sulfonamide 7,7-dioxide is resolved with di-p-toluoyl-L-tartaric acid followed by neutralization of the chiral salt yields the compound (4S,6S)4-(ethylamino)-6-methyl-5,6-dihydro-4H-thieno[2,3-b]thiopyran-2-sulfonamide 7,7-dioxide, which on reaction with hydrochloric acid in isopropanol results in the required (4S,6S)4-(ethylamino)-6-methyl-5,6-dihydro-4H-thieno[2,3-b]thiopyran-2-sulfonamide 7,7-dioxide hydrochloride.

PCT patent publication WO2010061398 discloses the process for the preparation of dorzolamide hydrochloride, wherein the racemic 4-(ethylamino)-6-methyl-5,6-dihydro-4H-thieno[2,3-b]thiopyran-2-sulfonamide 7,7-dioxide is treated with maleic acid in water to get trans-dorzolamide maleate salt. The maleate salt is further neutralized and then resolution with di-p-toluoyl-L-tartaric acid followed by neutralization of the chiral salt yields (4S,6S)-4-(ethylamino)-6-methyl-5,6-dihydro-4H-thieno[2,3-b]thiopyran-2-sulfonamide 7,7-dioxide. The compound (4S,6S)-4-(ethylamino)-6-methyl-5,6-dihydro-4H-thieno[2,3-b]thiopyran-2-sulfonamide 7,7-dioxide on reaction with hydrochloric acid in isopropanol results in the required pure trans-dorzolamide hydrochloride.

PCT patent publication WO2011101704 and corresponding Indian Patent application 426/CHE/2010 describes the process for the preparation of trans-dorzolamide hydrochloride by forming the maleate salt of racemic 4-(ethylamino)-6-methyl-5,6-dihydro-4H-thieno[2,3-b]thiopyran-2-sulfonamide 7,7-dioxide. The maleate salt is further neutralized and then resolution with di-p-toluoyl-L-tartaric acid followed by neutralization of the chiral salt yields trans-(45,65)-4-(ethylamino)-6-methyl-5,6-dihydro-4H-thieno[2,3-b]thyopyran-2-sulfonamide 7,7-dioxide. The compound trans-(4S,6S)-4-{ethylamino)-6-methyl-5,6-dihydro-4H-thieno[2,3-b]thiopyran-2-sulfonamide 7,7-choxide on reaction with hydrochloric acid in isopropanol results in the required trans-{S,S)-dorzolamide hydrochloride.

S.M. Thomas et al., "In Process Tests for the Synthesis of Dorzolamide", Analytical Chemistry, 1997, vol. 2, pages 432-439, discloses in-process analytical methods that are intended to be used to control the production of dorzolamide hydrochloride using carbonic anhydrase inhibitors. Indian Patent application 3431/MUM/2012 discloses the process wherein racemic 4-(ethylamino)-6-methyl-5,6-dihydro-4H-thieno[2,3-b]thiopyran-2-sulfonamide 7,7-dioxide is resolved using dibenzoyl-L-tartaric acid monohydrate or di-p-toluoyl-L-tartaric acid monohydrate in methanol followed by neutralization of the chiral salt, and then the (4S,6S)-4-(ethylamino)-6-methyl-5,6-dihydro-4H-thieno[2,3-b]thiopyran-2-sulfonamide 7,7-dioxide thus obtained is treated with hydrochloric acid in isopropanol to result in the required trans-(S,S)-dorzolamide hydrochloride. The compound is further recrystallised in isopropanol to isolate pure dorzolamide hydrochloride.

The prior art processes disclosed as above have several drawbacks in the preparation of puretrans-dorzolamide hydrochloride viz.,
1. involves column chromatography for separation of the desired diastereomer;
2. involves preparation of corresponding diastereomeric salts and converting again to base before preparation and isolation of pure trans-dorzolamide hydrochloride;
3. involves additional step of reacting the racemic dorzolamide base with mineral acid to isolate corresponding dorzolamide salt which is again converted to dorzolamide base and further resolved using resolving agent to form the corresponding salt, neutralization and isolation of the chiral dorzolamide base before reacting with hydrochloric acid to obtain dorzolamide hydrochloride; and
4. involves an additional step of reacting the racemic dorzolamide base with carboxylic acid to isolate corresponding dorzolamide salt which is again converted to dorzolamide base and resolved using resolving agent to form the corresponding salt, neutralization and isolation of the chiral dorzolamide base before reacting with hydrochloric acid to obtain dorzolamide hydrochloride.

As is evident from the cursory review of the prior arts that the preparation of pure dorzolamide hydrochloride involves either column chromatography for isolation of trans-isomer followed by use of resolving agent or involves repeated preparations of chiral or diastereomeric salts, use of resolving agent followed by converting into dorzolamide base and then isolating pure dorzolamide hydrochloride devoid of the diastereomeric impurity of cis-isomer.

Therefore, there remai ns a need in the art to develop a simple and cost effective process for the preparation of dorzolamide hydrochloride which ameliorates the above drawbacks of the prior arts and makes the process industrially viable and economically advantageous. The present invention therefore seeks to address these issues by providing an improved and cost-effective process that can easily be scaled for industrial production of dorzolamide hydrochloride.

The present inventors have developed an alternative process for isolating pure dorzolamide hydrochloride substantially free from the cis-isomer without using the time consuming column chromatography technique, repeated preparation of chiral salts, diastereomeric salts and converting into base before hydrochloride salt formation to isolate pure trans-(S,S)-dorzolamide hydrochloride.

### OBJ ECTIVE OF THE INVENTION:

The object of the present invention is to provide a process for the preparation of pure dorzolamide hydrochloride substantially free from impurity of cis-isomer. Yet another objective of the present invention is to provide a process for the preparation of dorzolamide hydrochloride without using the chiral resolving agent to remove the cis-isomer.

Yet another objective of the present invention is to provide a process for the preparation of pure dorzolamide hydrochloride by using industrially safe, environment friendly and cost effective process avoiding the repeated acid base treatment of 4-(ethylamino)-6-methyl-5,6-dihydro-4H-thieno[2,3-b]thiopyran-2-sulfonamide 7,7-dioxide.

### SUMMARY OF THE INVENTION:

Accordingly, the present invention provides a process for preparing dorzolamide hydrochloride the compound of Formula I using industrially safe and cost effective process avoiding the repeated acid base treatment of 4-(ethylamino)-6-methyl-5,6-dihydro-4H-thieno[2,3-b]thiopyran-2-sulfonamide 7,7-dioxide. According to primary object of the present invention, there is provided a process for the preparation of dorzolamide hydrochloride the compound of Formula I, which comprises the steps of:
a. reacting (6S)-4-(ethylamino)-6-methyl-5,6-dihydro-4H-thieno[2,3-b]thiopyran-2-sulfonamide 7,7-dioxide with hydrochloric acid in the presence of solvent to isolate mixture of cis- and trans-isomer of crude (6S)-4-(ethylamino)-6-methyl-5,6-dihydro-4H-thieno[2,3-b]thiopyran-2-sulfonamide 7,7-dioxide hydrochloride;
b. treating the mixture of cis- and trans-isomer of crude (6S)-4-(ethylamino)-6-methyl-5,6-dihydro-4H-thieno[2,3-b]thiopyran-2-sulfonamide 7,7-dioxide hydrochloride with an organic solvent to isolate trans-(4S,6S)-4-(ethylamino)-6-methyl-5,6-dihydro-4H-thieno[2,3-b]thiopyran-2-sulfonamide 7,7-dioxide hydrochloride substantially free from cis-diastereomer impurity; and
c. treating the trans-(4S,6S)-4-(ethylamino)-6-methyl-5,6-dihydro-4H-thieno[2,3-b]thiopyran-2-sulfonamide 7,7-dioxide hydrochloride with a solvent to get pure (4S,6S)-4-(ethylamino)-6-methyl-5,6-dihydro-4H-thieno[2,3-b]thiopyran-2-sulfonamide 7,7-dioxide hydrochloride.

These and the other objects of the present invention will be apparent from the following detailed description.

### DETAILED DESCRITION OF THE INVENTION:

All the terms used in the present application, unless otherwise indicated, must be interpreted in their ordinary meaning as known in the field. Other more specific definitions for some terms used in the present application are given below and are intended to be applied uniformaly to the entire description and claims, unless otherwise indicated.

The term "substantially_ as used in the specification means the compound dorzolamide hydrochloride having the impurity of the cis-isomer less than 1.0% The term "substantially_ as used in the specification means the compound dorzolamide hydrochloride having the impurity of the cis-isomer preferably less than 0.5%.

The term "substantially_ as used in the specification means the compound dorzolamide hydrochloride having the impurity of the cis-isomer more preferably less than 0.2%.

The term 'racemic_ as used in the present specification means the compound having mixture of cis-isomer and trans-isomer in any ratio.

The present invention provides an improved process for the preparation of (4S,6S)-4-(ethylamino)-6-methyl-5,6-dihydro-4H-thieno[2,3-b]thiopyran-2-sulfonamide 7,7-dioxide hydrochloride of the Formula I, which comprises the steps of: 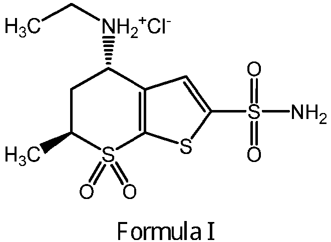
a. reacting (6S)-4-(ethylamino)-6-methyl-5,6-dihydro-4H-thieno[2,3-b]thiopyran-2-sulfonamide 7,7-dioxide with hydrochloric acid in the presence of solvent to isolate mixture of cis- and trans-isomer of crude (6S)-4-(ethylamino)-6-methyl-5,6-dihydro-4H-thieno[2,3-b]thiopyran-2-sulfonamide 7,7-dioxide hydrochloride;
b. treating the mixture of cis- and trans-isomer of crude (6S)-4-(ethylamino)-6-methyl-5,6-dihydro-4H-thieno[2,3-b]thiopyran-2-sulfonamide 7,7-dioxide hydrochloride with an organic solvent to isolate trans-(4S,6S)-4-(ethylamino)-6-methyl-5,6-dihydro-4H-thieno[2,3-b]thiopyran-2-sulfonamide 7,7-dioxide hydrochloride substantially free from cis-diastereomer impurity; and
c. treating the trans-(4S,6S)-4-(ethylamino)-6-methyl-5,6-dihydro-4H-thieno[2,3-b]thiopyran-2-sulfonamide 7,7-dioxide hydrochloride with a solvent to get pure (4S,6S)-4-(ethylamino)-6-methyl-5,6-dihydro-4H-thieno[2,3-b]thiopyran-2-sulfonamide 7,7-dioxide hydrochloride.

In an embodiment of the present invention the compound (6S)-4-(ethylamino)-6-methyl-5,6-dihydro-4H-thieno[2,3-b]thiopyran-2-sulfonamide 7,7-dioxide containing mixture of cis- and trans-diastereomer is converted to its corresponding pharmaceutically acceptable salt by reacting with hydrochloric acid at temperature range of 0éC to 5éC to isolate crude mixture of cis- and trans-(6S)-4-(ethylamino)-6-methyl-5,6-dihydro-4H-thieno[2,3-b]thiopyran-2-sulfonamide 7,7-dioxide hydrochloride.

In an embodiment of the present invention, the hydrochloric acid used for the preparation of pharmaceutically acceptable salt of the (6S)-4-(ethylamino)-6-methyl-5,6-dihydro-4H-thieno[2,3-b]thiopyran-2-sulfonamide 7,7-dioxide is gaseous hydrochloric acid and the pharmaceutically acceptable salt thus obtained is dorzolamide hydrochloride salt.

In an embodiment of the present invention, the reaction in step (a) is carried out in presence of solvent selected from the group consisting of methanol, isopropanol, acetone, methyl ethyl ketone, methyl isobutyl ketone, ethyl acetate, isopropyl acetate or a mixture thereof. In a preferred embodiment the solvent used for the preparation of hydrochloride salt is acetone.

In an embodiment of the present invention, in the reaction of step (b), the compound crude (6S)-4-(ethylamino)-6-methyl-5,6-dihydro-4H-thieno[2,3-b]thiopyran-2-sulfonamide 7,7-dioxide hydrochloride is treated with an organic solvent to isolate trans-(4S,6S)-4-(ethylamino)-6-methyl-5,6-dihydro-4H-thieno[2,3-b]thiopyran-2-sulfonamide 7,7-dioxide hydrochloride substantially free from cis-diastereomer impurity.

In an embodiment of the present invention, the organic solvent used for the removal of cis-diastereomer impurity is selected from the group consisting of linear or branched C₁ to C₄ alcohols comprising of methanol, ethanol, propanol, isopropanol, n-butanol, sec-butanol, tert-butanol and water or a mixture thereof. In a preferred embodiment the solvent used for the removal of cis-diastereomer impurity is selected from methanol and water or mixture thereof.

In an embodiment of the present invention, the organic solvent used for the removal of cis-diastereomer impurity is methanol.

In an embodiment of the present invention, the reaction of step (b) is carried out at a suitable temperature, preferably at a temperature ranging from 30éC to the reflux temperature of the solvent used for the reaction. In a preferred embodiment, the reaction is carried out at 50éC to 70éC.

In an embodiment of the present invention, the reaction of step (b) is carried out at a temperature in the range of 55éC to 65éC.

In an embodiment of the present invention, the compound trans-(6S)-4-(ethyl ami no)-6-methyl-5,6-di hydro-4H -thi eno[2,3-b]thiopyran-2-sulfonami de 7,7-dioxide hydrochloride substantially free from cis-diastereomer impurity obtained from step (b) is further purified using single or mixture of solvents selected from the group consisting of methanol, isopropanol, acetone, methyl ethyl ketone, methyl isobutyl ketone, and water or a mixture thereof. In a preferred embodiment the solvent used for the purification is mixture of solvent acetone and water.

In an embodiment of the present invention, the purification in the step (c) is carried out by making the slurry of the trans-(4S,6S)-4-(ethylamino)-6-methyl-5,6-dihydro-4H-thieno[2,3-b]thiopyran-2-sulfonamide 7,7-dioxide hydrochloride in the preferred solvent at the temperature in the range of 50éC to 60éC.

The process of the present invention has several advantages over the prior art teachings because it avoids the use of time consuming column chromatography technique, repeated preparation of chiral salts, diastereomeric salt and conversion of the salts into base before hydrochloride salt formation. Further the process has additional advantage of isolating pure trans-(4S,6S)-4-(ethylamino)-6-methyl-5,6-dihydro-4H-thieno[2,3-b]thiopyran-2-sulfonamide 7,7-dioxide hydrochloride which is substantially free from the cis-diastereomer impurity. Since the present inventors have employed the solvent treatment technique throughout the process steps which renders the process very economi cal and simple to implement on the industrial scale.

The following examples, which fully illustrate the practice of the preferred embodiments of the present invention, are intended to be for illustrative purpose only, and should not be considered to be limiting to the scope of the present invention.

### Examples:

### Example 1: Preparation of (6S)-4-(ethylamino)-6-methyl-5,6-dihydro-4H-thieno[2,3-b]thiopyran-2-sulfonamide 7,7-dioxide hydrochloride:

In a dry flask charged (6S)-4-(ethylamino)-6-methyl-5,6-dihydro-4H-thieno[2,3-b]thiopyran-2-sulfonamide 7,7-dioxide (50.0 gm) in acetone (700 ml) under stirring and cooled to 0éC. Maintaining the temperature at 0éC to 5éC purged hydrochloric acid gas to adjust the pH to acidic between the range of 1-2. After attaining desired pH, maintained the reaction mass for two hours at 0éC to 5éC under stirring. Filtered the precipitated compound (6S)-4-(ethylamino)-6-methyl-5,6-dihydro-4H-thieno[2,3-b]thiopyran-2-sulfonamide 7,7-dioxide hydrochloride and washed the solid mass with chilled acetone (50 ml). Dried the compound at 60-65éC till constant weight.
Dry weight: 50 g
HPLC purity: 77.62% [cis-isomer: 22.11%]

### Example 2: Preparation of trans-(4S,6S)-4-(ethylamino)-6-methyl-5,6-dihydro-4H-thieno[2,3-b]thiopyran-2-sulfonamide 7,7-dioxide hydrochloride [C rude dorzolamide hydrochloride]:

In a dry flask charged (6S)-4-(ethylamino)-6-methyl-5,6-dihydro-4H-thieno[2,3-b]thiopyran-2-sulfonamide 7,7-dioxide hydrochloride (19.0 g) and methanol (190 ml) at temperature of 25éC to 30éC. Under stirring raised the temperature of the reaction mass to reflux and maintained at reflux temperature for a period of two hours. After maintaining cooled the reaction mass gradually to 10éC to 15éC. Filtered the compound trans-(4S,6S)-4-(ethylamino)-6-methyl-5,6-dihydro-4H-thieno[2,3-b]thiopyran-2-sulfonamide 7,7-dioxide hydrochloride and washed the solid mass with chilled methanol. Dried the compound at 60-65éC till constant weight.
Dry weight: 12.8 g
HPLC purity: 99.33% [cis-isomer: 0.5%]

### Example 3: Purification of trans-(4S,6S)-4-(ethylamino)-6-methyl-5,6-dihydro-4H-thieno[2,3-b]thiopyran-2-sulfonamide7,7-dioxide hydrochloride:

In a dry flask charged trans-(4S,6S)-4-(ethylamino)-6-methyl-5,6-dihydro-4H-thieno[2,3-b]thiopyran-2-sulfonamide 7,7-dioxide hydrochloride (11.0 g), acetone (11 ml) and purified water (5.5 ml) at the temperature of 25éC to 30éC. Raised the temperature of the reaction slurry to reflux and maintained at reflux for one hour. Diluted the reaction mass with fresh acetone (44 ml) maintaining the temperature at reflux and continued maintaining at reflux temperature further for one hour. Cooled the reaction mass gradually to 10éC to 15éC and maintained. Filtered the compound pure (4S,6S)-4-(ethylamino)-6-methyl-5,6-dihydro-4H-thieno[2,3-b]thiopyran-2-sulfonamide 7,7-dioxide hydrochloride solid mass and washed the pure compound with chilled acetone (11 ml). Dried at 55éC to 60éC till constant weight.
Dry weight: 8.8 g
HPLC purity: 99.89% [cis-isomer: not detected]
[Total impurities: 0.11%]

### Example 4: Preparation of trans-(4S,6S)-4-(ethylamino)-6-methyl-5,6-dihydro-4H-thieno[2,3-b]thiopyran-2-sulfonamide 7,7-dioxide hydrochloride [C rude dorzolamide hydrochloride]:

Charged (6S)-4-(ethylamino)-6-methyl-5,6-dihydro-4H-thieno[2,3-b]thiopyran-2-sulfonamide 7,7-dioxide hydrochloride (5.0 g), methanol (22.5 ml) and 2.5 ml purified water at temperature of 25éC to 30éC. Under stirring raised the temperature of the reaction mass to reflux and maintained at reflux temperature for a period of two hours. After maintaining cooled the reaction mass gradually to 30éC to 35éC. Filtered the solid compound trans-(4S,6S)-4-(ethylamino)-6-methyl-5,6-dihydro-4H-thieno[2,3-b]thiopyran-2-sulfonamide 7,7-dioxide hydrochloride and washed with methanol (10 ml). Dried the compound at 60éC to 65éC till constant weight.
Dry weight: 3.2 g
HPLC purity: 99.47% [cis-isomer: 0.43%]

## Claims

1. A process for preparation of (4S,6S)-4-(ethylamino)-6-methyl-5,6-dihydro-4H-thieno[2,3-b]thiopyran-2-sulfonamide 7,7-dioxide hydrochloride, the compound of Formula I, 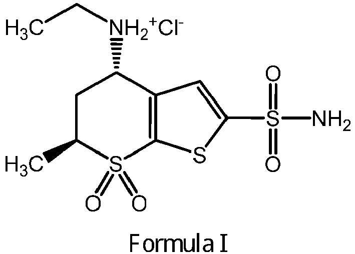 which comprises the steps of;
a. reacting (6S)-4-(ethylamino)-6-methyl-5,6-dihydro-4H-thieno[2,3-b]thiopyran-2-sulfonamide 7,7-dioxide with hydrochloric acid in the presence of solvent to isolate mixture of cis- and trans-isomer of (6S)-4-(ethylamino)-6-methyl-5,6-dihydro-4H-thieno[2,3-b]thiopyran-2-sulfonamide 7,7-dioxide hydrochloride;
b. treating the mixture of cis- and trans-isomer of (6S)-4-(ethylamino)-6-methyl-5,6-dihydro-4H-thieno[2,3-b]thiopyran-2-sulfonamide 7,7-dioxide hydrochloride with an organic solvent to isolate (4S,6S)-4-(ethylamino)-6-methyl-5,6-dihydro-4H-thieno[2,3-b]thiopyran-2-sulfonamide 7,7-dioxide hydrochloride substantially free from cis-diastereomer impurity; and
c. treating the (4S,6S)-4-(ethylamino)-6-methyl-5,6-dihydro-4H-thieno[2,3-b]thiopyran-2-sulfonamide 7,7-dioxide hydrochloride with a solvent to get pure (4S,6S)-4-(ethylamino)-6-methyl-5,6-dihydro-4H-thieno[2,3-b]thiopyran-2-sulfonamide 7,7-dioxide hydrochloride the compound of Formula I.

2. The process according to claim 1, wherein, the reaction of step (a) is carried out in the presence of solvent selected from the group consisting of methanol, isopropanol, acetone, methyl ethyl ketone, methyl isobutyl ketone, ethyl acetate, isopropyl acetate or a mixture thereof.

3. The process according to claim 1, wherein, the reaction of step (a) the hydrochloric acid is gaseous hydrochloric acid.

4. The process according to claim 1, wherein, the reaction of step (b) is carried out in the presence of solvent selected from the group consisting of linear or branched C₁ to C₄ alcohol comprising of methanol, ethanol, propanol, isopropanol, n-butanol, sec-butanol, tert-butanol and water or a mixture thereof.

5. The process according to claim 1, wherein, the reaction of step (b) is carried out in the presence of a solvent selected from methanol, water or a mixture thereof.

6. The process according to claim 1, wherein, the reaction of step (b) is carried out at a temperature in the range of 50°C to70°C.

7. The process according to claim 1, wherein, the reaction of step (c) is carried out in presence of a solvent selected from the group of methanol, isopropanol, acetone, methyl ethyl ketone, methyl isobutyl ketone, and water or a mixture thereof.

8. The process according to claim 1, wherein, the reaction of step (c) is conducted in presence of mixture of acetone and water.

9. The process according to claim 1, wherein, the reaction of step (c) is carried out at a temperature in the range of 50°C to 60°C.

## Patentansprüche

1. Verfahren zur Herstellung von (4S,6S)-4-(Ethylamino)-6-methyl-5,6-dihydro-4H-thieno[2,3-b]thiopyran-2-sulfonamid-7,7-dioxid-Hydrochlorid, der Verbindung von Formel I: 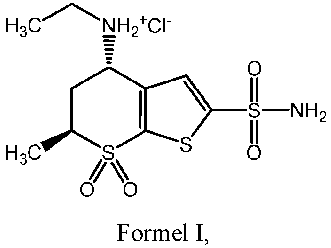 das die folgenden Schritte umfasst:
a. Reagieren von (6S)-4-(Ethylamino)-6-methyl-5,6-dihydro-4H-thieno[2,3-b]thiopyran-2-sulfonamid-7,7-dioxid mit Salzsäure in Gegenwart von Lösungsmittel, um die Mischung aus cis- und trans-Isomer von (6S)-4-(Ethylamino)-6-methyl-5,6-dihydro-4H-thieno[2,3-b]thiopyran-2-sulfonamid-7,7-dioxid-Hydrochlorid zu isolieren;
b. Behandeln der Mischung aus cis- und trans-Isomer von (6S)-4-(Ethylamino)-6-methyl-5,6-dihydro-4H-thieno[2,3-b]thiopyran-2-sulfonamid-7,7-dioxid-Hydrochlorid mit einem organischen Lösungsmittel, um (4S,6S)-4-(Ethylamino)-6-methyl-5,6-dihydro-4H-thieno[2,3-b]thiopyran-2-sulfonamid-7,7-dioxid-Hydrochlorid im Wesentlichen frei von Verunreinigung an cis-Diastereomer zu isolieren; und
c. Behandeln des (4S,6S)-4-(Ethylamino)-6-methyl-5,6-dihydro-4H-thieno[2,3-b]thiopyran-2-sulfonamid-7,7-dioxid-Hydrochlorids mit einem Lösungsmittel, um reines (4S,6S)-4-(Ethylamino)-6-methyl-5,6-dihydro-4H-thieno[2,3-b]thiopyran-2-sulfonamid-7,7-dioxid-Hydrochlorid, die Verbindung der Formel I, zu erhalten.

2. Verfahren nach Anspruch 1, wobei die Reaktion von Schritt (a) in Gegenwart von Lösungsmittel durchgeführt wird, das aus der Gruppe ausgewählt ist, die aus Folgenden besteht: Methanol, Isopropanol, Aceton, Methylethylketon, Methylisobutylketon, Ethylacetat, Isopropylacetat oder einer Mischung davon.

3. Verfahren nach Anspruch 1, wobei in der Reaktion von Schritt (a) die Salzsäure gasförmige Salzsäure ist.

4. Verfahren nach Anspruch 1, wobei die Reaktion von Schritt (b) in Gegenwart von Lösungsmittel durchgeführt wird, das aus der Gruppe ausgewählt ist, die aus Folgenden besteht: linearem oder verzweigtem C₁- bis C₄-Alkohol, der Methanol, Ethanol, Propanol, Isopropanol, n-Butanol, sec-Butanol, tert-Butanol umfasst, und Wasser oder einer Mischung davon.

5. Verfahren nach Anspruch 1, wobei die Reaktion von Schritt (b) in Gegenwart eines Lösungsmittels durchgeführt wird, das aus Methanol, Wasser oder einer Mischung davon ausgewählt ist.

6. Verfahren nach Anspruch 1, wobei die Reaktion von Schritt (b) bei einer Temperatur in dem Bereich von 50 °C bis 70 °C durchgeführt wird.

7. Verfahren nach Anspruch 1, wobei die Reaktion von Schritt (c) in Gegenwart eines Lösungsmittels durchgeführt wird, das aus der Gruppe von Methanol, Isopropanol, Aceton, Methylethylketon, Methylisobutylketon und Wasser oder einer Mischung davon ausgewählt ist.

8. Verfahren nach Anspruch 1, wobei die Reaktion von Schritt (c) in Gegenwart einer Mischung aus Aceton und Wasser ausgeführt wird.

9. Verfahren nach Anspruch 1, wobei die Reaktion von Schritt (c) bei einer Temperatur in dem Bereich von 50 °C bis 60 °C durchgeführt wird.

## Revendications

1. Procédé de préparation du chlorhydrate de (4S,6S)-4-(éthylamino)-6-méthyl-5,6-dihydro-4H-thiéno[2,3-b]thiopyran-2-sulfonamide 7,7-dioxyde, le composé de Formule I, 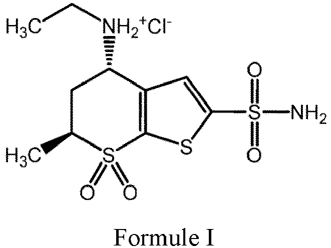 qui comprend les étapes :
a. de réaction de (6S)-4-(éthylamino)-6-méthyl-5,6-dihydro-4H-thiéno[2,3-b]thiopyran-2-sulfonamide 7,7-dioxyde avec de l'acide chlorhydrique en présence de solvant afin d'isoler un mélange d'isomères cis et trans du chlorhydrate de (6S)-4-(éthylamino)-6-méthyl-5,6-dihydro-4H-thiéno[2,3-b]thiopyran-2-sulfonamide 7,7-dioxyde ;
b. de traitement du mélange d'isomères cis et trans du chlorhydrate de (6S)-4-(éthylamino)-6-méthyl-5,6-dihydro-4H-thiéno[2,3-b]thiopyran-2-sulfonamide 7,7-dioxyde par un solvant organique afin d'isoler le chlorhydrate de (4S,6S)-4-(éthylamino)-6-méthyl-5,6-dihydro-4H-thiéno[2,3-b]thiopyran-2-sulfonamide 7,7-dioxyde sensiblement exempt d'impuretés de diastéréoisomère cis ; et
c. de traitement du chlorhydrate de (4S,6S)-4-(éthylamino)-6-méthyl-5,6-dihydro-4H-thiéno[2,3-b]thiopyran-2-sulfonamide 7,7-dioxyde par un solvant afin d'obtenir le chlorhydrate de (4S,6S)-4-(éthylamino)-6-méthyl-5,6-dihydro-4H-thiéno[2,3-b]thiopyran-2-sulfonamide 7,7-dioxyde pur, le composé de Formule I.

2. Procédé selon la revendication 1, dans lequel la réaction de l'étape (a) est effectuée en présence d'un solvant choisi dans le groupe constitué par le méthanol, l'isopropanol, l'acétone, la méthyléthylcétone, la méthylisobutylcétone, l'acétate d'éthyle, l'acétate d'isopropyle, ou un mélange de ceux-ci.

3. Procédé selon la revendication 1, dans lequel, dans la réaction de l'étape (a), l'acide chlorhydrique est l'acide chlorhydrique gazeux.

4. Procédé selon la revendication 1, dans lequel la réaction de l'étape (b) est effectuée en présence d'un solvant choisi dans le groupe constitué par un alcool en C₁-C₄ linéaire ou ramifié comprenant le méthanol, l'éthanol, le propanol, l'isopropanol, le n-butanol, le sec-butanol, le tertio-butanol, et l'eau, ou un mélange de ceux-ci.

5. Procédé selon la revendication 1, dans lequel la réaction de l'étape (b) est effectuée en présence d'un solvant choisi parmi le méthanol, l'eau, ou un mélange de ceux-ci.

6. Procédé selon la revendication 1, dans lequel la réaction de l'étape (b) est effectuée à une température dans la plage allant de 50°C à 70°C.

7. Procédé selon la revendication 1, dans lequel la réaction de l'étape (c) est effectuée en présence d'un solvant choisi dans le groupe constitué par le méthanol, l'isopropanol, l'acétone, la méthyléthylcétone, la méthylisobutylcétone, et l'eau, ou un mélange de ceux-ci.

8. Procédé selon la revendication 1, dans lequel la réaction de l'étape (c) est effectuée en présence d'un mélange d'acétone et d'eau.

9. Procédé selon la revendication 1, dans lequel la réaction de l'étape (c) est effectuée à une température dans la plage allant de 50°C à 60°C.
